# EUROPEAN PATENT APPLICATION

(11) **EP 4 763 478 A1**
(43) Date of publication of application: **24.06.2026**
(21) Application number: 24222982.1
(22) Date of filing: 23.12.2024
(51) Int. Cl.: B29C 64/106, B29C 48/05, B29C 48/25, B33Y 10/00, B33Y 30/00, B29C 64/241, B29C 64/336

(54) **METHOD OF MANUFACTURING A JACKET ASSEMBLY AND MATERIAL EXTRUSION MACHINE FOR MANUFACTURING A JACKET ASSEMBLY**

(71) Applicant: Creganna Unlimited Company, Ballybrit, Galway H91 VN2T (IE)
(72) Inventor: McDermott, Bernard, Galway, H91 VN2T (IE); Kaftanski, Piotr, Galway, H91 VN2T (IE)
(74) Representative: Grünecker Patent- und Rechtsanwälte PartG mbB

(57) **Abstract**

The present invention relates to a method of manufacturing a jacket assembly, with the following steps: providing a shaft (102), the shaft (102) having a length (a); applying an extrudate (117) using a material extrusion, MEX, machine (112), the MEX machine (112) having a gantry (115) that comprises at least one head (114); depositing the extrudate (117) via the at least one head (114) on at least a part of the shaft (102); moving the shaft (102) such that the extrudate (117) provides a jacket (106) on at least a part of the shaft (102); and varying a type of the extrudate (117) while depositing the extrudate (117).

Further, the present invention also relates to a material extrusion, MEX, machine (112) for manufacturing a jacket assembly that is configured to deposit an extrudate (117) via at least one head (114).

## Description

The present disclosure relates to a method of manufacturing a jacket assembly and to a material extrusion machine for manufacturing the jacket assembly.

The assembly of polymer jackets during a manufacturing process as for example for catheter devices is generally a manual process and can be difficult and time-consuming.

A catheter is a medical device, generally a thin wall tube constructed from medical grade materials, which may be inserted into the body and guided through a lumen such as a blood vessel, trachea and airways, urinary or gastrointestinal tract to treat disease or perform a medical procedure. Catheters are designed and manufactured for specific applications such as endovascular, urological, gastrointestinal, neurovascular, and ophthalmic procedures.

Catheter devices are typically constructed through a multi-layer assembly process, beginning with a mandrel over which a liner and a reinforcement is added. Over this, a jacket layer of thermoplastic polymer is applied, which is then consolidated using a reflow process to create a composite structure with defined flexibility properties.

Catheter devices generally require a transition in flexibility from a relatively stiff proximal end to a relatively flexible distal end. This is typically achieved by various means, one being by changing the hardness of the jacket material or the type of the jacket material in steps or continuously from the proximal to the distal end.

Achieving the desired flexibility gradient from a stiffer proximal end to a more flexible distal end involves varying the jacket material's hardness or composition. Different sections of jacket extrusions are cut to specific lengths and manually assembled onto the shaft in a defined order determined by the flexibility requirements before being reflowed using a heat shrink layer.

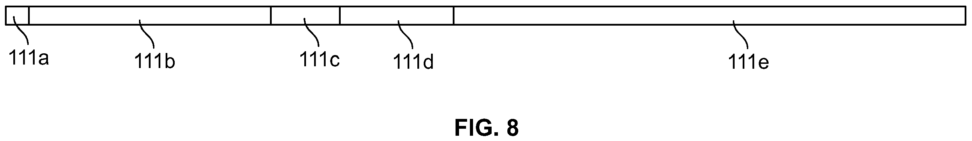

**Figure 8** shows a possible construction of a typical medical catheter having jacket sections 111a - 111e with multiple hardness and materials. Softer, low durometer jacket materials are especially difficult to assemble as they are generally tacky and tend to stick together and to the reinforced shaft during assembly. This tackiness also makes them prone to pick up foreign materials (FM) such as fibers and dust which are detrimental to final product quality. Misalignments of the jacket segments during reflow can cause flaws in the jacket coverage and this along with FM inclusions can result in reject assemblies.

Assembling such jackets requires highly skilled and trained personal and can be time consuming. Due to the amount of manual work, the quality of the jacket varies and the scrap rate can be high leading to high product costs.

It is therefore an object of the present invention to provide an improved manufacturing method for assembling a jacket and an improved material extrusion machine that reduces assembly time.

This problem is solved by the subject-matter of the independent claims. Advantageous examples of the present disclosure are the subject-matter of the dependent claims.

The present disclosure is based on the idea that a material extrusion (MEX) machine manufactures a jacket assembly by applying a jacket onto a shaft.

In particular, the present invention relates to a method of manufacturing a jacket assembly, the method having the following steps: providing a shaft, the shaft having a length; applying an extrudate using a material extrusion (MEX) machine, the MEX machine having a gantry that comprises at least one head; depositing the extrudate via the at least one head on at least a part of the shaft; moving the shaft such that the extrudate provides a jacket on at least a part of the shaft and varying a type of extrudate while depositing the extrudate.

This method advantageously provides a finely controlled jacket in an automated process leading to a reduced process time.

By printing or material extruding the jacket materials directly onto the surface of a sub-assembly, which could be a mandrel, a mandrel with an assembled liner layer or a mandrel, liner and assembled braided or coiled reinforcement layer, the manual assembly of the jacket materials can be eliminated. This method also eliminates the requirement for multiple polymer jacket extrusions of different material types and durometers and specific wall thicknesses for each specific catheter product, reducing raw material costs, part numbers and significant volumes of materials.

The present invention further relates to a material extrusion (MEX) machine for manufacturing a jacket assembly, with the MEX machine comprising: a gantry that comprises at least one head and at least one container for storing one type of feedstock. The at least one head is provided with the feedstock and the MEX machine is configured to form at least one type of extrudate from the one type of feedstock and configured to deposit the extrudate via the at least one head.

The present MEX machine works within small tolerances such that a high quality can be ensured over a high number of products. Further, the automated process reduces amount of manual work that is required, which reduces scrap rate. This leads to reduced product costs per piece.

According to an advantageous further development of the present disclosure, the method also comprises the step of: moving the shaft in a first axial direction while the extrudate is applied via the MEX machine. Moving the shaft axially while the jacket is applied facilitates the step of applying the jacket along the shaft's length. Exemplarily, the shaft can be moved via a 2D drive.

According to an advantageous further development of the present disclosure, the method further comprises the step of rotating the shaft in a radial direction around its longitudinal axis while the extrudate is applied via the MEX machine. This advantageously further easies the application of the extrudate. The rotational movement of the shaft together with the axial movement allows a smooth and fast depositing of the jacket in a spiral configuration. The rotation allows the extrudate to be applied to the complete circumference of the shaft.

In a further development of the present invention, the method also comprises the step of moving the gantry in the first axial direction while the extrudate is applied via the MEX machine or moving the gantry in a second axial direction while the extrudate is applied via the MEX machine, with the second axial direction being perpendicularto the first axial direction. This step of moving the gantry in one of the axial directions can be performed in addition or instead of the axial movement step of the shaft. Therefore the gantry is advantageously mounted on a rack along which either the first or the second axial movement is performed. Exemplarily, while the gantry moves along the rack, the shaft also moves along the axial direction. In addition, the shaft also rotates in a radial direction in order to apply the extrudate along the complete circumference of the shaft. The axial movement of the gantry and the shaft provides the advantage that the jacket can be applied over a longer length even when the movement of the gantry itself is limited. This enables a small and compact MEX machine.

It is to be noted that it is also possible that the shaft does not rotate, but the head of the MEX machine rotates around the circumference of the shaft in order to apply the filament along the complete circumference of the shaft. Further, in another example the shaft moves in the first axial direction, while the MEX machine rotates in a radial direction.

Advantageously, the present MEX directly applies materials such as thermoplastic materials onto a shaft in a rotary process. This creates a rotary based MEX machine capable of aiding in the assembly of products such as single or multi-material medical catheters and also cable jackets, sleeves, or other tubular based constructions or applications.

According to an advantageous further development of the present disclosure, the step of varying the type of extrudate comprises: applying a first segment of the jacket having a first type of extrudate and consecutively applying a second segment of the jacket having a second type of extrudate; the second type of extrudate being different from the first type of extrudate.

This provides an easy-solution to apply a jacket consisting of different extrudate types along the length of the shaft. It is clear that the jacket may be applied along the complete length of the shaft or only along parts of the shaft. The different extrudate types may form segments of the jacket. The segments can be directly placed next to each other or having a section without a jacket in between.

By forming consecutively the different segments of the jacket, a fast and repeatable manufacturing process is established. Such a solution makes the manual assembly of different jacket segments redundant. By automating this process, the scrap rate is drastically reduced.

According to an advantageous further development of the present disclosure, the jacket is provided by applying the extrudate as a spiral that winds around the shaft and/or advantageously the extrudate is a polymer material. The polymer material is preferably a thermoplastic polymer material i.e. becomes pliable or moldable above a specific temperature and solidifies upon cooling. The polymer material advantageously can be any type of polymer material. Various hardness levels can be used depending on the application. The hardness, which may also be referred to as "durometer", is intended to mean the shore D hardness, a parameter specific to the chosen polymer material.

According to an advantageous further development of the present disclosure, the method further comprises the steps of: providing a heatshrink along at least a part of the length of the shaft on top of the applied jacket and heating the heatshrink in order to consolidate the jacket and the shaft.

The applied heat advantageously merges the jacket with the shaft such that the different components and materials form a unified structure. Further, the used MEX machine works within small tolerances such that a high quality can be ensured over a high number of products.

According to an advantageous further development of the present disclosure, the extrudate is formed from a feedstock. The MEX machine head that is advantageously used to apply the jacket receives the feedstock and forms the extrudate that is applied as the output of the MEX machine head onto the shaft.

According to an advantageous further development of the present disclosure, the feedstock comprises a filament or the feedstock comprises pellets. The described manufacturing method can be used independently of the exact type of MEX machine. Either a filament or pellets can be used as an input material to each MEX head. The extrudate that is outputted from the head of the MEX machine then provides the basis for applying the jacket with the advantageous manufacturing method.

According to an advantageous further development of the present disclosure, the different types of extrudate are formed from different types of feedstock that vary in the material composition. Thereby, the material composition exemplarily can affect the hardness of the feedstock and therefore also the hardness of the extrudate. Thus, a jacket having different properties in different areas of the shaft can be provided along the length of the shaft. Besides the hardness of the material, the different types of extrudate may also vary in other material properties such as heat resistance, radiopacity, or lubricity colors. The feedstock may also be materials with additives to enhance particular properties such as for instance radiopacity, lubricity, stability and strength. The feedstock may also be in a variety of colors.

Advantageously, the MEX machine comprises at least two heads and the first segment of the jacket is applied via a first of the at least two heads and the second segment of the jacket is applied via a second of the at least two heads. In this advantageous manufacturing method, the different segments of jackets are applied by different heads of the machine. Therefore, each head of the machine advantageously deposits an extrudate having a type different from the type of extrudate deposited by the other head. The switch between the heads that are depositing the extrudate and thereby forming the segments of the jacket is performed by switching the head in the gantry. The different heads are stored in a docking station from which, the currently used, active head is provided at the gantry such that the corresponding extrudate can be applied on the shaft.

Advantageously the gantry is movable in an axial direction while the extrudate is deposited. Preferably, the gantry is mounted on a rack along which the gantry is movable. In an advantageous further development, the MEX machine comprises at least two heads and at least two containers each storing a different type of feedstock. Further, the MEX machine forms two types of extrudate from the two different types of feedstock and each of the at least two heads deposits a different type of extrudate. This MEX machine is exemplarily configured to deposit extrudate formed from feedstock that comprises a filament or pellets.

To better understand the present disclosure, this is explained in greater detail using the example depicted in the following Figures. Identical parts are hereby provided with identical reference numbers and identical component names. Furthermore, some features or combinations of features from the various examples shown and described may also represent independent solutions, inventive solutions or solutions according to the disclosure. In the drawings:
- **Fig. 1**: shows a schematic representation of a material extrusion machine for manufacturing a jacket assembly according to a first example of the present invention;
- **Fig. 2A**: shows a cross-sectional representation of the jacket assembly according to the present invention;
- **Fig. 2B**: shows a cross-sectional representation of the jacket assembly after the reflow process;
- **Fig. 3**: shows a further schematic representation of a material extrusion machine for manufacturing a jacket assembly according to a second example of the present invention;
- **Fig. 4**: shows a further view on the material extrusion machine according to the second example of the present invention;
- **Fig. 5**: shows a further schematic representation of a material extrusion machine for manufacturing a jacket assembly according to a third example of the present invention;
- **Fig. 6**: shows a further schematic representation of a material extrusion machine for manufacturing a jacket assembly according to a fourth example of the present invention;
- **Fig. 7**: shows a further schematic representation of a material extrusion machine for manufacturing a jacket assembly according to a fifth example of the present invention;
- **Fig. 8**: shows a schematic representation of a known jacket assembly having different sections.

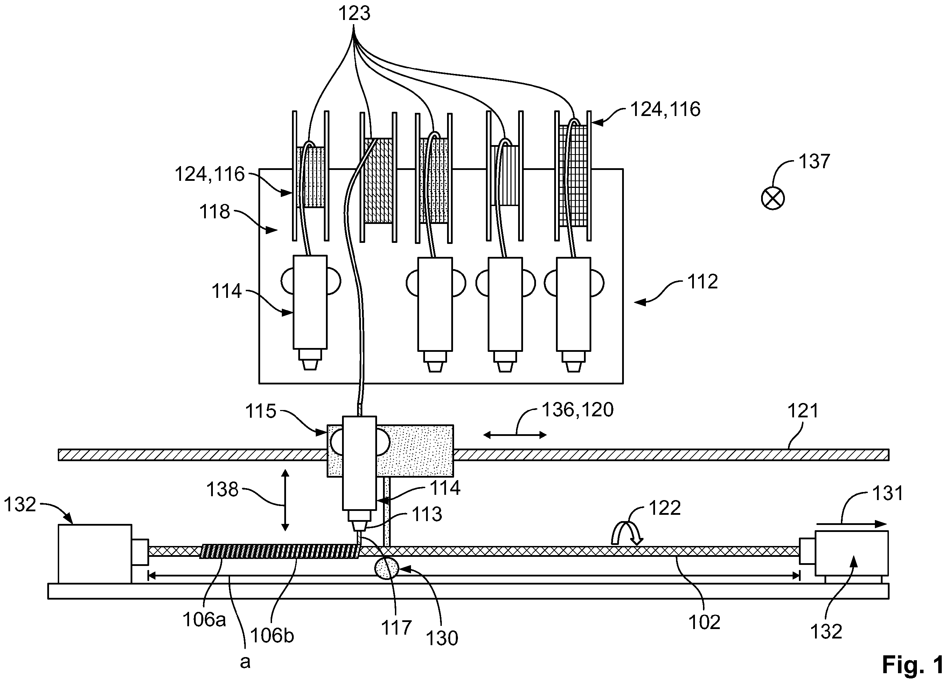

The present invention will now be explained in more detail with reference to the Figures and firstly referring to Fig. 1.

The presented MEX machine aims to deposit a layer onto a shaft, whereby the material and properties of the deposited layer can be varied. This allows to deposit a layer that has different properties and comprises different types of material along the complete length of the deposited layer. The deposited layer can also be a single material type along the length of the shaft. In the following, different examples of MEX machines all allowing to execute the same advantageous method are presented.

**Fig. 1** shows a schematic representation of a material extrusion (MEX) machine for manufacturing a jacket assembly according to a first example of the present invention. The present method is advantageously used to assembly a jacket that is preferably used in the medical field.

The MEX machine 112 according to a first example comprises a docking station 118 with exemplarily five MEX heads 114. The heads 114 can be in the in form of a printer.

Each head 114 is provided with feedstock 123 from a container 124. The machine 112 advantageously comprises five containers 124 that can be placed in a docking station 118. Each of the five containers 124 stores a different type of feedstock 123. Thus, in this example five types of feedstock 123 can be deposited with the MEX machine.

Each container 124 is exemplarily depicted as a filament spool 116 with feedstock that comprises filament. Each of the filament spool 116 in this example provides the filament to one individual head 114. It is clear that any other number of containers, feedstock and heads are within the scope of the present invention. Exemplarily, each filament fed to one head 114 has its own specific durometer, type or compound of polymer. Meaning that exemplarily the different types of feedstock 123 vary in properties of the material such as hardness, radiopacity, color, lubricity or strength.

The MEX machine 112 advantageously forms five types of extrudate 117 from the five types of feedstock 123. Meaning that from one type of feedstock one type of extrudate 117 is formed and whenever another type of feedstock 123 is provided another type of extrudate 117 is formed. The extrudate 117 is deposited via a nozzle 113 of the head 114.

The MEX machine 112 can be equipped with various heads 114 with differing nozzle geometries from circular to rectangular in order to change the deposition rate of extrudate. For instance, a rectangular nozzle will allow a wide bead to be applied and this will allow an increase of pitch of the spiral of applied polymer, thereby increasing the speed of application.

The varying between the different heads while depositing the extrudate is performed in a fast manner such that the depositing process may continue without large interruptions or stops. What type of extrudate is deposited can be flexibly defined depending on the application.

Preferably, the MEX machine 112 deposits the extrudate 117 that forms a layer, jacket 106, on a shaft 102. The shaft 102 extends in an x-direction 136 and is gripped at both ends by rotary drive chucks 132 that rotate the shaft in a radial direction 122 and control the corresponding rotational speed. By rotating the shaft 102 while the MEX machine 112 deposits the extrudate 117, the extrudate is easily deposited along the complete circumference of the shaft 102. Further, the shaft 102 is positioned and tensioned between the two rotatory chucks 132 by a tensioning mechanism 131.

Further, the MEX machine 112 exemplarily includes the gantry 115 that is arranged on a rack 121. In operation, the gantry 115 holds at least one head 114, which is fed with feedstock 123. The gantry 115 is movable along the rack 121 in a first axial direction 120 while the extrudate 117 is deposited. In this example, the x-direction 136 coincides with the first axial direction 120. In that way, the gantry 115 traverses over the length a of the shaft in the first axial direction 120. The position of the gantry 115 allows the docking station 118 to remain fixed in the same position, while allowing the machine to deposit the extrudate 117 along the length a of the shaft.

The traverse movement of the gantry 115 can be bi-directional such that the gantry 115 may move back and forth along the length a of the shaft 102. Further, the height of the nozzle 113 of the heads 114 can be also be controlled and varied in a z-direction 138 such that the distance from the nozzle 113 to the shaft 102 varies.

Preferably, the gantry 115 moves in the first axial direction 120 back and forth and the shaft 102 rotates in the radial direction 122 while the extrudate 117 is deposited in a spiral configuration. Thereby, the rotational speed of the shaft is controlled and matched to the transverse movement of the gantry 115 in the first axial direction 120.

The tensioning mechanism 131 preferably tensions the shaft 102 in order to reduce potential movement of the shaft during the rotation of the shaft. In such an example, the jacket 106 is provided by applying the extrudate 117 as a spiral that winds around the shaft 102. Depending on the rotational and axial movement of the shaft and the gantry, the pitch of the spiral can be varied.

Once a section of a particular extrudate is printed, the extrudate 117 is stopped and the head 114 is exchanged for another material head 114 and the process is repeated for each required extrudate, laying down each material adjoining the previous segment or interleaved with the previous segment or overlapping the previous segment. In this way, multiple materials or durometers and arrangements of jacket materials can be applied to the shaft 102. The length of one segment of jacket having one type of extrudate 117 can be varied and differed for each segment.

As a result, the jacket 106 comprises one segment 106a having a first type of extrudate 117 and one segment 106b having a second type of extrudate 117. Preferably, the two segments 106a, 106b are arranged adjacent to each other in a consecutive manner. The two types of extrudates may vary in the exact material composition and therefore in the exact properties and behavior. Further, different types of extrudate may have further varying properties.

It is clear that the jacket 106 may comprise more than two segments each being formed from a different type of extrudate 117. Further, the jacket 106 is not necessarily applied along the complete length a of the shaft, but rather on at least a part of the length a. The jacket 106 may also comprise a single segment formed from one type of extrudate 117 deposited from one head only.

The extrudate that is formed from the feedstock preferably comprises a polymer material. The polymeric material is preferably a thermoplastic polymer material i.e. becomes pliable or moldable above a specific temperature and solidifies upon cooling. The polymer material advantageously can be any type of polymer material. Various hardness levels can be used depending on the application. The hardness, which may also be referred to as "durometer", is intended to mean the shore D hardness, a parameter specific to the chosen polymer material. Each segment of jacket 106 may have a different durometer value such that the jacket provides a varying support for the shaft 102.

To further easy the depositing process, a support unit 130 is arranged below the depositing head 114. The support unit 130 moves together with the head 114 or together with the shaft 102 such that the support unit preferably stays in vicinity to the position where the extrudate is applied onto the shaft 102. The support unit 130 preferably supports the shaft and prevents bowing or whipping of the shaft and keeps the position of the nozzle 113 over the shaft 114 within limits with respect to height and centering over the shaft.

As shown in the above described first example of the present invention, the gantry 115 and/or the shaft 102 move in a first axial direction 120 along an x-direction 136. The movement or switch of the heads 114 from the position in the docking station 118 to the active position in the gantry 115 is performed along a z-direction 138. The corresponding y-direction 137 in this example is arranged along the plane of projection.

Further examples of MEX machine that are within the scope of the present invention having a configuration with some components arranged in a different direction are described further below.

The method how the extrudate is deposited that forms the jacket remains the same and some detailed steps might be omitted in the following examples for brevity and readability.

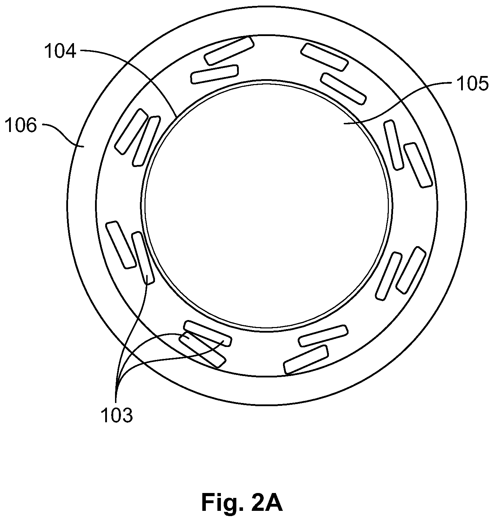

**Fig. 2A** shows an exemplarily cross-sectional view of a shaft 102. The shaft 102 in this example comprises a mandrel 105 covered with a liner 104 and overlaid with a braided reinforcement layer 103. The jacket 106 in such an example is deposited on top of the reinforcement layer 103. The cross-sectional view shows one example of a shaft 102 configuration with applied jacket 106.

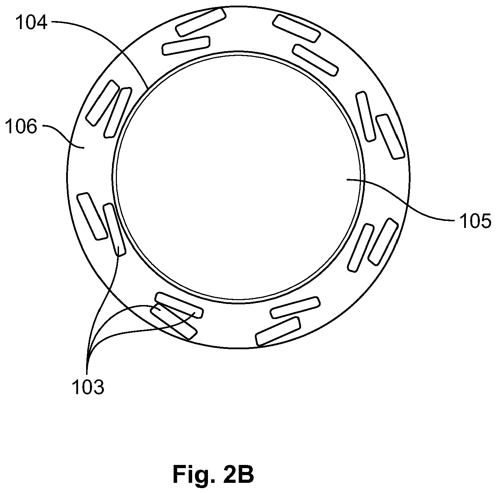

However, the presented method and machine works independent of the exact shaft configuration onto which the jacket is deposited. To consolidate the applied jacket 106, a heatshrink (not shown) may be arranged on top of the jacket 106. By heating the heatshrink, the heatshrink contracts radially while melting the jacket material 106. The radial contraction forces the jacket material 106 to reflow into position and to form a layer that encloses the shaft 102. The reflowed jacket 106 is shown in **Fig 2B****.** Afterwards, the heatshrink 108 is removed again. Other methods of consolidation are of course also applicable.

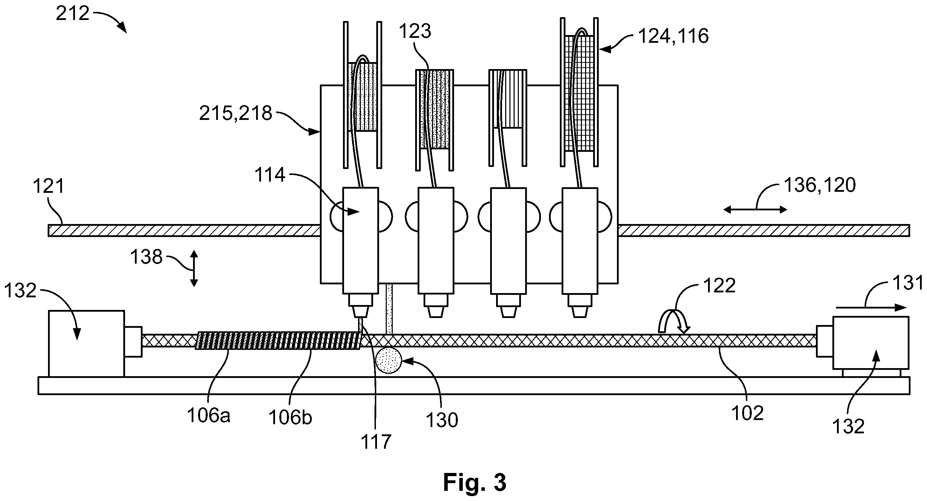

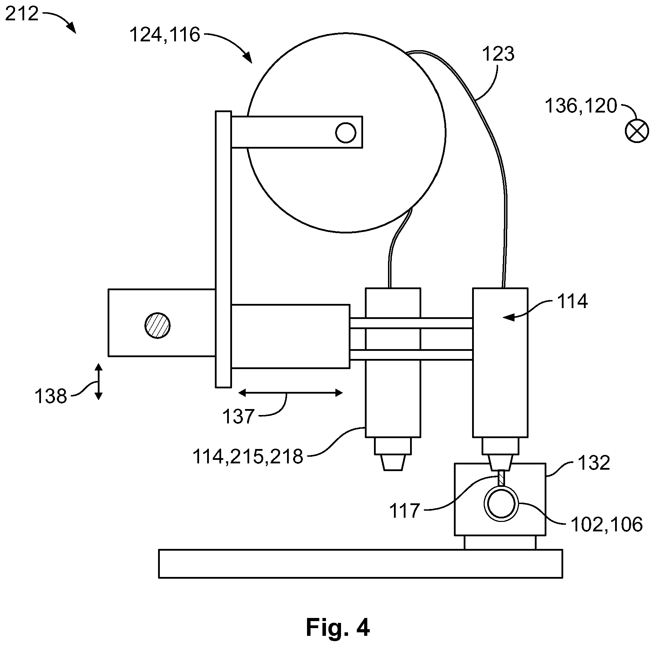

**Fig. 3** **and** **Fig 4** show a schematic representation of a MEX machine 212 according to a second example of the present invention.

The method to deposit a jacket onto a shaft as described with respect to the first example in Fig. 1 can also be performed with the MEX machine 212 as described in the following. Steps or components for which no detailed adapted description is mentioned perform and work the same way as described with respect to Fig.1. The same applies for the later following examples.

In particular, as can be seen in Fig. 3 and Fig 4, the MEX machine 212 differs in the arrangement of the docking station 218 and the gantry 215. In this example, the gantry 215 is comprised in the docking station 218. During the deposition of the jacket, an MEX head 114 is selected from the gantry 215 and moved forward into position over the shaft 102 with a defined height in the z-direction 138 above the shaft 102. Moving forward in this scenario means that the selected head 114 moves in the plane of projection (y-direction 137) to a position above the shaft 102 in the z-direction 138. The other heads 114, which are here exemplarily shown as three further heads 114, are arranged in the gantry 215 that is positioned in the plane projection behind the center of the shaft 102. The shaft 102 again extends in the x-direction 136.

This can be seen by the side view shown in Fig. 4. Only one head 114 is positioned centered above the shaft 102, whereas the other heads 114 are positioned in the docking station/gantry 218/215 in a y-direction 137 behind the shaft 102. The MEX machine 212 also allows multiple heads to be moved forward to deposit the extrudate simultaneously at different positions along the length a of the shaft 102. In this way, different segments of the jacket 106 formed from extrudate from different types can be applied at the same time. This advantageously increases the speed of jacket application.

Further, the complete docking station 218 / gantry 215 is arranged at the rack 121 and can move in the first axial direction 120 that extends along the x-direction 136 during the depositing process.

Again, the shaft 102 may rotate in the radial direction 122 while the gantry 215 with the heads 114 moves in the first axial direction 120 to deposit the jacket 106 at required parts along the length a of the shaft 102.

Here, the feedstock 123 is exemplarily shown as filament that is stored in containers 124 in the form of filament spools 116. Each filament spool 116 exemplarily feeds one head 114 with a type of filament from which the extrudate 117 is formed.

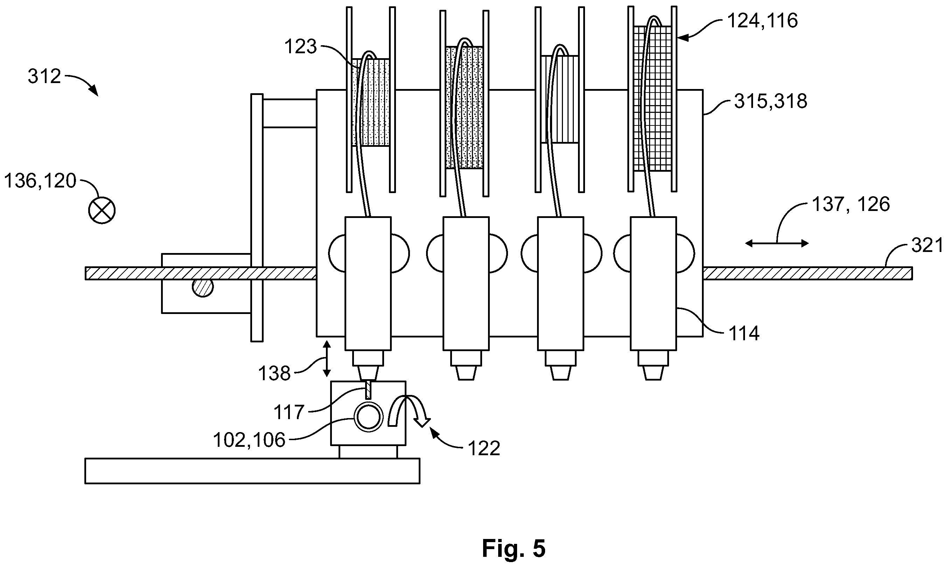

A third example of the MEX machine is shown in **Fig. 5****.** The MEX machine 312 advantageously performs the same method steps and has the same features as described before, but extends along different axes than the other examples.

Again, the length of the shaft 102 extends in an x-direction 136 that in this Figure extends along the plane projection. The distance between the nozzle 113 of the head 114 and the point of the shaft 102 where the extrudate 117 is deposited is still denoted as the z-direction 138.

An additional rack 321 in this example extends along the y-direction 137, perpendicular to the x direction 136. Along the rack 321, the docking station 318 that exemplarily comprises the gantry 315 is positioned. Thus, in comparison to the MEX machine as shown with respect to Fig. 1 and 3, the position of the gantry and the heads has been rotated by 90 °.

In the machine configuration as shown in Fig. 5, only one head is positioned above the shaft 102 at a given point in time. Only the head 114 that actively deposits the extrudate 117 is positioned above the shaft 102. The gantry 315 is movable along a second axial direction 126 to switch between different heads 114. In this example, the second axial direction 126 extends along the y-direction 137.

The shaft 102 advantageously rotates in the radial direction 122. The gantry 315 can also move in the first axial direction 120.

By moving in the second axial direction 126, the gantry 315 positions a head 114 over the centerline of the shaft 102. As the shaft rotates, the gantry 315 and the head 114 move in the first axial direction 120 thereby extruding a bead of material onto the shaft 102 in a spiral configuration.

Again, the different heads 114 are exemplarily fed with different types of feedstock 123 such that each head 114 deposits a different type of extrudate 117 onto the shaft 102.

Exemplarily, the feedstock 123 comprises filament that is stored in the docking station 318 in containers 124 formed as filament spools 116.

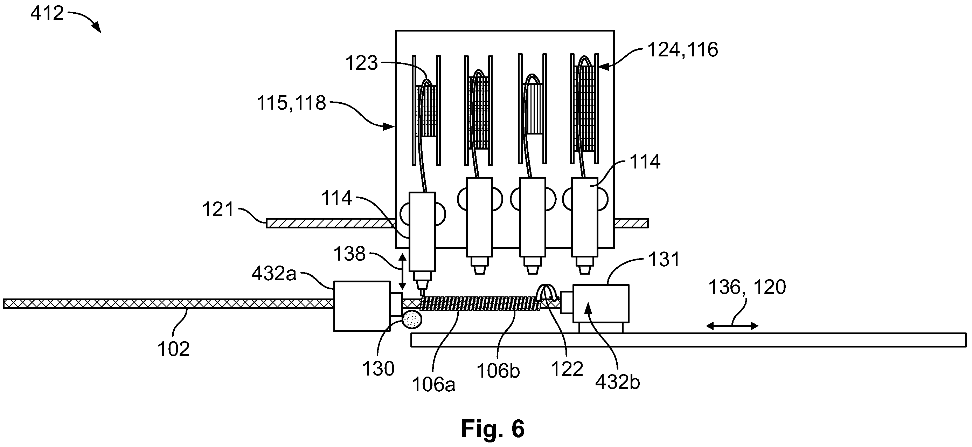

**Fig. 6** shows a MEX machine 412 according to a fourth example. The shaft 102 is again arranged along the x-direction 136, as well as the rack 121 and the docking station 118. In this example, the gantry 115 is comprised in the docking station 118. The heads 114 in the gantry 115 are again arranged along the x-direction 136.

Also in this example, at least one head 114 is arranged in the gantry 115. Fig. 6 shows exemplarily four heads. The gantry 115 may move along the first axial direction 120, which here exemplarily extends in the x-direction 136, along the rack 121 in order to bring the heads into the correct position above the shaft 102 to deposit the extrudate 117.

Further, the shaft 102 may rotate in the radial direction 122. The rotation is performed by a rotary drive chuck 432b. A rotary guide bushing support 432a is provided on the opposite side, which stays stationary, while the rotary drive chuck 432b rotates the shaft 122 and moves the shaft 102 in the first axial direction 120 back and forth while the extrudate 117 is deposited via one of the heads 114. Thus, the shaft 102 is rotated in the radial direction 122 and moved back and forth in order to allow the extrudate 117 to form the jacket 106 in a spiral configuration at the required position along the length a of the shaft. The axial movement of the shaft 102 may be performed together with the axial movement of the gantry 115 to allow longer shafts being deposited with extrudate 117 even when the rack 121 has a reduced length. The position of the gantry 115 allows the docking station 118 to remain fixed in the same position, while allowing the machine to deposit the extrudate 117 along the length a of the shaft.

The distance between the head 114 that is active and deposits the extrudate 117 and the outer circumference of the shaft 102 can be varied.

Again, the extrudate is formed from different type of feedstock, which feed different heads. An exact description thereof can be found with respect to the other examples of the MEX machine and are also applicable for this example.

As can be seen in Fig. 6, also with the MEX machine 412 according to the fourth example, a jacket 106 comprising multiple segments, here exemplarily two segments 106a, 106b, can be deposited. Thereby, each segment advantageously is formed from a different type of extrudate having varying material properties.

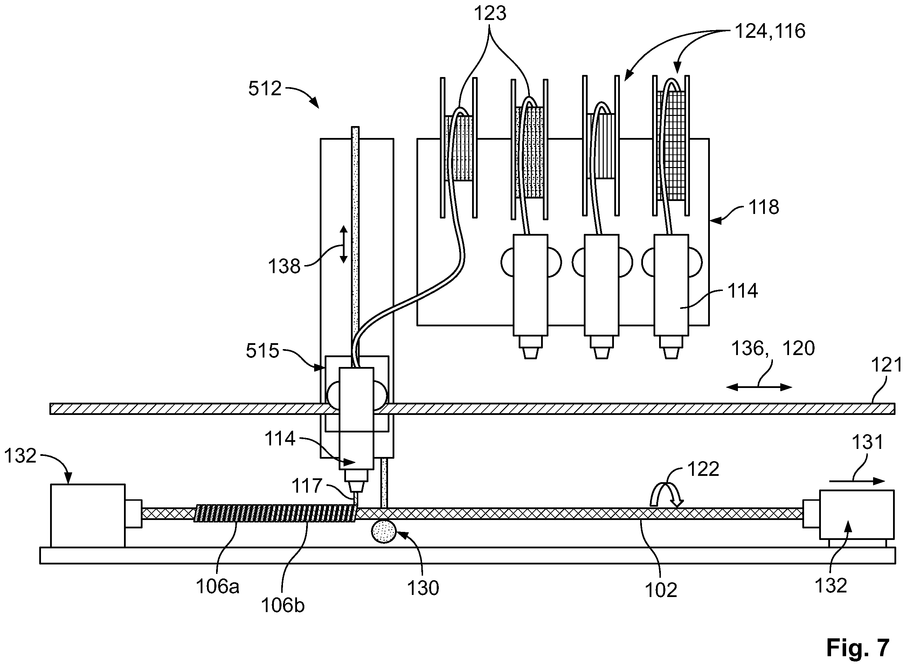

**Fig. 7** shows the MEX machine according to a fifth example of the present invention. The MEX machine 115 comprises the gantry 515 arranged on the rack 121 that extends along the x-direction 136. The gantry 515 in this example is in the form of a tool changing mechanism. In this example, exemplarily four heads 114 are stored in the docking station 118. The head 114 that deposits the extrudate 117 onto the shaft 102 is moved from the docking station 118 to the tool changing mechanism 515 via which the head moves along the rack 121. Via the tool changing mechanism 515, the active head 114 that deposits the extrudate 117 is quickly changed with another head from the docking station 118.

Again, the shaft 102 can rotate in the rotational direction 122 while the extrudate 117 is deposited. The advantageous MEX machine 512 also enables to deposit a jacket 106 onto the shaft 102 formed from extrudates 117 having a different material type. Each head 114 is exemplarily shown as being fed with feedstock 123 from different containers 124.

The containers are exemplarily shown as filament spools 116 that feed a filament to the head from which the extrudate is formed.

The tool changing mechanism 515 may involve various methods to secure the working head 114 including but not limited to mechanical coupling, electro-mechanical coupling, motor driven interlocking, electro-magnetic interlocking, and use of pneumatics and hydraulics actuated devices for locking. The head 114 and tool changing mechanism 515 will move back and forth in the first axial direction 120 in order to deposit the extrudate along the length a of the shaft 102. The applied jacket 106 may only be formed at parts of the length of the shaft 102.

The method that is performed by all described MEX machines comprises the following steps.

The method comprises the steps of providing a shaft 102, which has a length a. A layer of extrudate 117 is applied to the shaft 102 using a MEX machine 112, 212, 312, 412, 512. The MEX machine having a gantry 115, 215, 315, 515 that comprises at least one head 114 via which the extrudate 117 is deposited on the shaft 102. Thereby, the extrudate 117 is deposited onto at least a part of the shaft 102. Further, the shaft 102 is moved such that the extrudate 117 provides a jacket 106 on at least a part of the shaft 102. While the extrudate 117 is deposited, the type of extrudate 117 is varied.

The extrudate can be varied by firstly providing one type of feedstock to the head and then manually changing the type of feedstock that is stored in the container and provided to the head. Further, different types of extrudate may also be deposited by having a machine with multiple heads each fed with one type of feedstock and by changing the head via which the extrudate is deposited. This alternative is described further above.

Thus, the manufacturing method according to the present invention enables to apply a jacket 106 onto at least a part of the shaft 102. The jacket 106 is formed by the extrudate 117.

Further, either the shaft and/or the gantry can be moved in the first axial direction 120, while the extrudate is applied via the MEX machine. Further, the shaft may be rotated in the radial direction 122 while the extrudate is applied via the MEX machine. Lastly, the gantry can also be moved in a second axial direction 126 that is perpendicular to the first axial direction 120.

Further, the movement of the gantry and the shaft is performed by precision linear stages with closed-loop positioning capability and with a positioning resolution of at least 1/10th the extrusion width or desired feature size. One or more heads can work on the shaft simultaneously to deposit the jacket material in sequence or concurrently. The machine can be controlled by a PLC or any electronics capable of motion control with adequate digital-analog IO, required components for temperature control, and queue-based motion planner and command look ahead.

### List of reference signs

| **Reference Numeral** | **Description** |
|---|---|
| 100 | Catheter assembly |
| 102 | Shaft |
| 103 | Reinforcement |
| 104 | Liner |
| 105 | Mandrel |
| 106 | Jacket |
| 106a | Segment of jacket of a first type |
| 106b | Segment of jacket of a second type |
| 111a-111e | Jacket sections |
| 112,212,312,412,512 | MEX machine |
| 113 | Nozzle |
| 114 | Head |
| 115,215,315,515 | Gantry |
| 116 | Filament spool |
| 117 | Extrudate |
| 118,218,318 | Docking station |
| 120 | First axial direction |
| 121,321 | Rack |
| 122 | Radial direction |
| 123 | Feedstock |
| 124 | Container |
| 126 | Second axial direction |
| 130 | Support unit |
| 131 | Tensioning mechanism |
| 132,432b | Rotary drive chuck |
| 432a | Rotary guide bushing support |
| a | Length of the catheter shaft |
| 136 | x direction |
| 137 | y direction |
| 138 | z direction |

## Claims

1. Method of manufacturing a jacket assembly, the method comprising the following steps:
- providing a shaft (102), the shaft (102) having a length (a);
- applying an extrudate (117) using a material extrusion, MEX, machine (112), the MEX machine (112) having a gantry (115) that comprises at least one head (114);
- depositing the extrudate (117) via the at least one head (114) on at least a part of the shaft (102);
- moving the shaft (102) such that the extrudate (117) provides a jacket (106) on at least a part of the shaft (102); and
- varying a type of the extrudate (117) while depositing the extrudate (117).

2. Method of manufacturing a jacket assembly according to claim 1, further comprising the step of:
moving the shaft (102) in a first axial direction (120) while the extrudate (117) is applied via the MEX machine (112).

3. Method of manufacturing a jacket assembly according to one of the preceding claims, further comprising the step of:
rotating the shaft (102) in a radial direction (122) while the extrudate (117) is applied via the MEX machine (112).

4. Method of manufacturing a jacket assembly according to one of the preceding claims, further comprising the step of:
moving the gantry (115) in the first axial direction (120) while the extrudate (117) is applied via the MEX machine (112) or
moving the gantry (115) in a second axial direction (126) while the extrudate (117) is applied via the MEX machine (112);
the second axial direction (126) being perpendicular to the first axial direction (120).

5. Method of manufacturing a jacket assembly according to one of the preceding claims,
wherein the step of varying the type of extrudate (117) comprises
applying a first segment (106a) of the jacket (106) having a first type of extrudate (117) and consecutively applying a second segment (106b) of the jacket (106) having a second type of extrudate (117);
the second type of extrudate (117) being different from the first type of extrudate (117).

6. Method of manufacturing a jacket assembly according to one of the preceding claims,
wherein the jacket (106) is provided by applying the extrudate (117) as a spiral that winds around the shaft (102) and/or wherein the extrudate (117) is a polymer material.

7. Method of manufacturing a jacket assembly according to one of the preceding claims, further comprising the steps of:
providing a heatshrink along at least a part of the length (a) of the shaft (102) on top of the applied jacket (106);
heating the heatshrink in order to consolidate the jacket (106) and the shaft (102).

8. Method of manufacturing a jacket assembly according to one of the preceding claims, wherein the extrudate (117) is formed from a feedstock (123).

9. Method of manufacturing a jacket assembly according to claim 8, wherein the feedstock (123) comprises a filament or wherein the feedstock (123) comprises pellets.

10. Method of manufacturing a jacket assembly according one of the preceding claims,
wherein the different types of extrudate (117) are formed from different types of feedstock (123) that vary in the material composition.

11. Method of manufacturing a jacket assembly according to claim 5 ,
wherein the MEX machine (112) comprises at least two heads (114) and
wherein the first segment (106a) of the jacket (106) is applied via a first of the at least two heads (114),
and the second segment (106b) of the jacket (106) is applied via a second of the at least two heads (114).

12. Material extrusion, MEX, machine (112) for manufacturing a jacket assembly,
the MEX machine (112) comprises:
a gantry (115) that comprises at least one head (114);
at least one container (124) for storing one type of feedstock (123);
wherein the at least one head (114) is provided with the feedstock (123);
wherein the MEX machine (112) is configured to form at least one type of extrudate (117) from the one type of feedstock (123) and is configured to deposit the extrudate (117) via the at least one head (114).

13. Material extrusion, MEX, machine (112) according to claim 12 ,
wherein the gantry (115) is movable in an axial direction (120,126) while the extrudate (117) is deposited.

14. Material extrusion, MEX, machine (112) according to one of claims 12 or 13,
wherein the MEX machine (112) comprises at least two heads (114) and at least two containers (124) each storing a different type of feedstock (123);
wherein the MEX machine (112) forms two types of extrudate (117) from the two different types of feedstock (123) and
each of the at least two heads (114) deposits a different type of extrudate (117).

15. Material extrusion, MEX, machine (112) according to one of claims 12 to 14,
wherein the MEX machine (112) is configured to deposit extrudate (117) formed from feedstock (123) that comprises a filament or pellets..
